# EUROPEAN PATENT APPLICATION

(11) **EP 4 091 644 A1**
(43) Date of publication of application: **23.11.2022**
(21) Application number: 21741074.5
(22) Date of filing: 13.01.2021
(51) Int. Cl.: A61L 33/06, C08J 7/04

(54) **POLYURETHANE OR POLYURETHANE UREA, ANTITHROMBOGENIC COATING AGENT, ANTITHROMBOGENIC MEDICAL TOOL, AND PRODUCTION METHOD**

(30) Priority: 17.01.2020 JP 2020005904
(71) Applicant: DKS Co. Ltd., Kyoto-shi, Kyoto 600-8873 (JP)
(72) Inventor: NISHIMURA Takuma, Kyoto-shi, Kyoto 600-8873 (JP); NISHIURA Masahito, Kyoto-shi, Kyoto 600-8873 (JP)
(74) Representative: TBK
(86) International application number: PCT/JP2021/000773
(87) International publication number: WO 2021/145330

(57) **Abstract**

Provided is a technology for exhibiting good antithrombogenicity.

A polyurethane or polyurethane urea has a graft chain having a specific structure. Two functional groups included in the specific structure are each an alkyl group having 1 to 20 carbon atoms, an aryl group having 6 to 12 carbon atoms, or an aralkyl group having 7 to 20 carbon atoms and may be the same or different from each other.

## Description

### Technical Field

The present invention relates to a polyurethane or polyurethane urea, an antithrombogenic coating agent, an antithrombogenic medical tool, and a production method.

### Background Art

It has been known that polyurethanes and polyurethane ureas are used as materials used for medical devices such as an artificial kidney, an artificial lung, a syringe, and a blood bag (for example, PTL 1). It is desirable that when such materials used for these medical devices come in contact with blood, the blood does not coagulate. In other words, the materials used for these medical devices preferably have good antithrombogenicity.

PTL 1 discloses, as a material used for medical devices, a polyurethane or polyurethane urea having a phosphorylcholine structure in a side chain thereof for the purpose of improving antithrombogenicity.

### Citation List

### Patent Literature

PTL 1: International Publication No. 1998/046659

### Summary of Invention

### Technical Problem

However, the polyurethane or polyurethane urea disclosed in PTL 1 does not have sufficient antithrombogenicity, and thus there has been room for improvement.

### Solution to Problem

The present invention has been made in order to solve the above problem and can be realized as the following aspects.

(1) According to an aspect of the present invention, there is provided a polyurethane or polyurethane urea. This polyurethane or polyurethane urea has a graft chain including a structure represented by general formula (1). 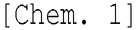 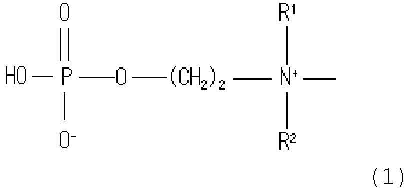 (In the formula, R¹ and R² are each an alkyl group having 1 to 20 carbon atoms, an aryl group having 6 to 12 carbon atoms, or an aralkyl group having 7 to 20 carbon atoms and may be the same or different from each other.)

The polyurethane or polyurethane urea of this aspect can exhibit good antithrombogenicity.

(2) In the polyurethane or polyurethane urea of the above aspect, the polyurethane or polyurethane urea may contain phosphorus derived from the structure represented by general formula (1) in an amount of 2.0 mmol or more and 10.0 mmol or less relative to 1.0 g of the polymer.

The polyurethane or polyurethane urea of this aspect can exhibit good mechanical strength as well as good antithrombogenicity.

(3) In the polyurethane or polyurethane urea of the above aspect, R¹ and R² may each be an alkyl group having 1 to 20 carbon atoms and may be the same or different from each other.

The polyurethane or polyurethane urea of this aspect can exhibit better antithrombogenicity.

(4) According to another aspect of the present invention, there is provided an antithrombogenic coating agent containing the polyurethane or polyurethane urea of the above aspect.

The antithrombogenic coating agent of this aspect can exhibit good antithrombogenicity.

(5) According to another aspect of the present invention, there is provided an antithrombogenic medical tool including the polyurethane or polyurethane urea of the above aspect.

The antithrombogenic medical tool of this aspect can exhibit good antithrombogenicity.

(6) According to another aspect of the present invention, there is provided a method for producing the polyurethane or polyurethane urea of the above aspect. This production method includes a step of, while a compound having the structure represented by general formula (1) and a functional group that reacts with a radical is brought into contact with a polyurethane or polyurethane urea, irradiating a surface of the polyurethane or polyurethane urea with ionizing radiation to generate a radical, thereby forming a graft chain.

The production method of this aspect enables the production of a polyurethane or polyurethane urea that exhibits good antithrombogenicity.

(7) According to another aspect of the present invention, there is provided a method for producing the polyurethane or polyurethane urea of the above aspect. This production method includes a step of irradiating a surface of a polyurethane or polyurethane urea with ionizing radiation to generate a radical, and a step of bringing a compound having the structure represented by general formula (1) and a functional group that reacts with a radical into contact with the surface to form a graft chain.

The production method of this aspect enables the production of a polyurethane or polyurethane urea that exhibits good antithrombogenicity.

### Description of Embodiments

### <Polyurethane or Polyurethane Urea>

A polyurethane or polyurethane urea according to an embodiment of the present invention has a graft chain including a structure represented by general formula (1) below. Hereinafter, a polyurethane and a polyurethane urea are also collectively referred to as a "polyurethane or the like". The "structure represented by general formula (1)" is also referred to as a "choline hydrogen phosphate structure".

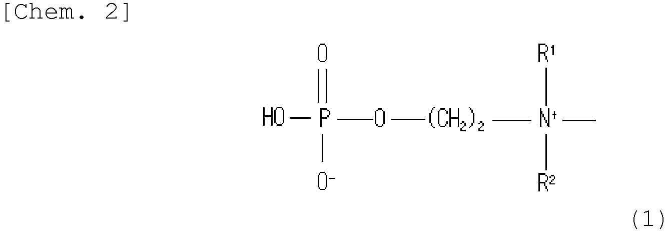

In general formula (1), R¹ and R² are each an alkyl group having 1 to 20 carbon atoms, an aryl group having 6 to 12 carbon atoms, or an aralkyl group having 7 to 20 carbon atoms and may be the same or different from each other.

The polyurethane or the like of this embodiment has good antithrombogenicity. The term "antithrombogenicity" as used herein refers to the property that, in the case of contact with blood, the blood is less likely to coagulate. Although the mechanism of good antithrombogenicity of the polyurethane or the like of this embodiment is not clear, the following mechanism is presumed. Specifically, the choline hydrogen phosphate structure is a structure similar to phosphatidylcholine, which forms a biological membrane, and has a feature of having, unlike a phosphorylcholine group, a hydroxyl group derived from a phosphate group at a terminal thereof. Since the polyurethane or the like of this embodiment has a hydroxyl group derived from a phosphate group at a terminal thereof, presumably, the interaction with water in the living body is enhanced, and as a result, the polyurethane or the like can exhibit good antithrombogenicity.

In addition, the polyurethane or the like of this embodiment has a graft chain including a choline hydrogen phosphate structure. That is, the polyurethane or the like has a choline hydrogen phosphate structure in a side chain of the resin composition. In general, since the main chain of a macromolecule is entangled, if a functional group that exhibits a performance is present in the main chain, the performance of the functional group is less likely to be sufficiently exhibited. In contrast, if a functional group that exhibits a performance is present in a side chain, presumably, the performance of this functional group is not suppressed by the main chain and thus is sufficiently exhibited. That is, since the polyurethane or the like of this embodiment has a graft chain including a choline hydrogen phosphate structure, the polyurethane or the like can sufficiently exhibit biocompatibility due to the hydroxyl group derived from the phosphate group and thus can probably exhibit good antithrombogenicity.

From the viewpoint of improving antithrombogenicity, R¹ and R² are each preferably an alkyl group having 1 to 20 carbon atoms or an aryl group having 6 to 12 carbon atoms, more preferably an alkyl group having 1 to 20 carbon atoms, still more preferably an alkyl group having 1 to 10 carbon atoms, and even more preferably an alkyl group having 1 to 5 carbon atoms.

The phosphorus content derived from the choline hydrogen phosphate structure is preferably 0.03 mmol or more and 20.0 mmol or less, more preferably 0.1 mmol or more and 10.0 mmol or less, and still more preferably 2.0 mmol or more and 10.0 mmol or less relative to 1.0 g of the polyurethane or the like of this embodiment. When the phosphorus content is within the preferred range, good antithrombogenicity can be exhibited, and good mechanical strength can also be exhibited.

### <Polyol>

A polyol used in the polyurethane or the like of this embodiment is not particularly limited, and, for example, publicly known polyols can be used. Examples of polyols include hydroxyl group-containing conjugated diene polymers and hydrogenated products thereof, castor oil, castor oil polyols and hydrogenated products thereof, polyether polyols, polyester polyols, polycaprolactone polyols, and polycarbonate polyols. The term "castor oil polyols" as used herein refers to polyols obtained by modifying castor oil and having hydroxyl groups at the terminals thereof.

Examples of hydroxyl group-containing conjugated diene polymers and hydrogenated products thereof include, but are not particularly limited to, hydroxyl group-containing conjugated diene polymers such as polybutadiene polyol and polyisoprene polyol, and hydrogenated products obtained by hydrogenating these polymers.

Examples of castor oil polyols and hydrogenated products thereof include, but are not particularly limited to, castor oil-modified polyols obtained by using castor oil or castor oil fatty acid as a raw material, and hydrogenated products thereof. Examples of such castor oil-modified polyols include, but are not particularly limited to, transesterification reaction products of castor oil and fat or oil other than castor oil, transesterification reaction products of castor oil and a fat/oil fatty acid, transesterification reaction products of castor oil and a polyhydric alcohol, esterification reaction products of castor oil fatty acid and a polyhydric alcohol, esterification reaction products of some of hydroxyl groups included in castor oil and a monocarboxylic acid such as acetic acid, reaction products obtained by subjecting any of these to addition polymerization of an alkylene oxide, and hydrogenated products obtained by hydrogenating any of these.

Examples of polyether polyols include, but are not particularly limited to, those obtained by subjecting a polyhydric alcohol to addition polymerization of an alkylene oxide. Examples of polyester polyols include, but are not particularly limited to, esterification reaction products of a polyhydric alcohol and a polyvalent carboxylic acid. Examples of polycaprolactone polyols include, but are not particularly limited to, those obtained by subjecting caprolactone to ring-opening polymerization. Examples of polycarbonate polyols include, but are not particularly limited to, reaction products of a polyhydric alcohol and a carbonic acid derivative such as diphenyl carbonate, dimethyl carbonate, or phosgene.

Among the above polyols, polyols having a structure with high hydrophobicity are preferred from the viewpoint of improving water resistance of the polyurethane or the like after graft polymerization. Specifically, polyether polyols obtained by subjecting a polyhydric alcohol to addition polymerization of an alkylene oxide having 3 or more carbon atoms, hydroxyl group-containing conjugated diene polymers and hydrogenated products thereof, polyester polyols which are esterification reaction products of a polyhydric alcohol having 4 or more carbon atoms and a polyvalent carboxylic acid having 5 or more carbon atoms, and polytetramethylene glycol are preferred, and polybutadiene polyol and polyester polyols which are esterification reaction products of a polyhydric alcohol having 4 or more carbon atoms and a polyvalent carboxylic acid having 5 or more carbon atoms are more preferred. These polyols may be used alone or in combination of two or more thereof.

The hydroxyl value of the polyol is not particularly limited, and is preferably 10 mgKOH/g or more and 200 mgKOH/g or less, more preferably 20 mgKOH/g or more and 150 mgKOH/g or less, and still more preferably 30 mgKOH/g or more and 120 mgKOH/g or less. The term "hydroxyl value" as used herein refers to the number of milligrams (mg) of potassium hydroxide required to neutralize acetic acid bonded to hydroxyl groups when 1 g of a sample is acetylated, and is measured in accordance with JIS K 0070-1992.

The average molecular weight of the polyol is not particularly limited, and is preferably 500 or more and 5,000 or less, and more preferably 800 or more and 4,000 or less.

### <Polyisocyanate>

A polyisocyanate used in the polyurethane or the like of this embodiment is not particularly limited, and, for example, publicly known polyisocyanates can be used. Examples of polyisocyanates include aliphatic polyisocyanates, alicyclic polyisocyanates, aromatic polyisocyanates, and araliphatic polyisocyanates.

Examples of aliphatic polyisocyanates include, but are not particularly limited to, tetramethylene diisocyanate, dodecamethylene diisocyanate, hexamethylene diisocyanate (hereinafter also referred to as "HDI"), 2,2,4-trimethylhexamethylene diisocyanate, 2,4,4-trimethylhexamethylene diisocyanate, lysine diisocyanate, 2-methylpentane-1,5-diisocyanate, and 3-methylpentane-1,5-diisocyanate.

Examples of alicyclic polyisocyanates include, but are not particularly limited to, isophorone diisocyanate, hydrogenated xylylene diisocyanate, 4,4'-dicyclohexylmethane diisocyanate, 1,4-cyclohexane diisocyanate, methylcyclohexylene diisocyanate, and 1,3-bis(isocyanatomethyl)cyclohexane.

Examples of aromatic polyisocyanates include, but are not particularly limited to, tolylene diisocyanate, 2,2'-diphenylmethane diisocyanate, 2,4'-diphenylmethane diisocyanate, 4,4'-diphenylmethane diisocyanate, (hereinafter also referred to as "MDI"), polymethylene polyphenyl polyisocyanate, 4,4'-dibenzyl diisocyanate, 1,5-naphthylene diisocyanate, 1,3-phenylene diisocyanate, and 1,4-phenylene diisocyanate.

Examples of araliphatic polyisocyanates include, but are not particularly limited to, xylylene diisocyanate (hereinafter also referred to as "XDI"), dialkyldiphenylmethane diisocyanate, tetraalkyldiphenylmethane diisocyanate, and α,α,α,α-tetramethylxylylene diisocyanate.

In addition, modified products of the above organic polyisocyanates may be used as the polyisocyanate. Examples of modified products of organic polyisocyanates include, but are not particularly limited to, carbodiimides, allophanates, biurets, isocyanurates, and adducts. These polyisocyanates may be used alone or in combination of two or more thereof.

From the viewpoint of improving mechanical properties of films of the polyurethane or the like, hexamethylene diisocyanate (HDI) or 4,4'-diphenylmethane diisocyanate (MDI) is preferably used as the polyisocyanate. From the viewpoint of generating stable radicals upon irradiation with ionizing radiation, hexamethylene diisocyanate is more preferably used.

The equivalent ratio (NCO group/OH group, hereinafter also referred to as an "NCO index") of isocyanate groups (NCO groups) of a polyisocyanate to hydroxyl groups (OH groups) of a polyol and hydroxyl groups (OH groups) of a chain extender and a crosslinking agent is not particularly limited, and is preferably 0.5 or more and 3.0 or less, and more preferably 0.8 or more and 1.5 or less.

The free isocyanate group content of the polyisocyanate is not particularly limited, and is preferably 20% by mass or more and 70% by mass or less, more preferably 25% by mass or more and 65% by mass or less, and still more preferably 30% by mass or more and 60% by mass or less. When the content is within the preferred range, good moldability is exhibited.

The average molecular weight of the polyisocyanate is not particularly limited, and is preferably 100 or more and 400 or less, and more preferably 150 or more and 300 or less.

### <Polyamine>

the polyurethane or the like encompasses a polyurethane urea. The term "polyurethane urea" as used herein refers to a polyurethane having a urea bond generated by a chemical reaction between a polyisocyanate and a polyamine. Examples of polyamines used for polyurethane ureas include, but are not particularly limited to, ethylenediamine, propylenediamine, hexylenediamine, isophoronediamine, xylylenediamine, piperazine, diphenylmethanediamine, ethyltolylenediamine, diethylenetriamine, dipropylenetriamine, triethylenetetramine, tetraethylenepentamine, and polyetheramine. Examples of polyamines used in polyurethane ureas further include aromatic diamines such as diethyltoluenediamine.

### <Others>

Other materials may be added to the polyurethane or the like of this embodiment as long as the effects of the present invention are not impaired. Examples of the other materials include, but are not particularly limited to, chain extenders, crosslinking agents, and catalysts.

Examples of chain extenders include, but are not particularly limited to, ethylene glycol, diethylene glycol, 1,4-butanediol, 1,3-butanediol, 2,3-butanediol, 1,3-propylene glycol, 1,2-propylene glycol, 1,6-hexanediol, 3-methyl-1,5-pentanediol, and neopentyl glycol. Examples of crosslinking agents include, but are not particularly limited to, aminoplast compounds, epoxy compounds, carbodiimide compounds, glycerin, and trimethylol propane.

Examples of catalysts include, but are not particularly limited to, metal catalysts and amine-based catalysts. Examples of metal catalysts include, but are not particularly limited to, tin catalysts such as dibutyltin dilaurate, dioctyltin dilaurate, and dibutyltin dioctoate; lead catalysts such as lead octylate, lead octenoate, and lead naphthenate; and bismuth catalysts such as bismuth octylate and bismuth neodecanoate. Examples of amine-based catalysts include, but are not particularly limited to, tertiary amine compounds such as triethylenediamine.

### <Production Method>

The method for producing a polyurethane or the like is not particularly limited, and the polyurethane or the like can be produced by a publicly known method. In such a case, all raw materials of the polyurethane or the like may be caused to react with each other at the same time. Alternatively, for example, some of raw materials may be caused to react with each other, and the remaining raw material may then be caused to react with the resulting product. The method for providing a graft chain to a polyurethane or the like is not particularly limited, and the production can be performed by a publicly known method. The method for providing a graft chain to a polyurethane or the like may be, for example, a method in which a polyurethane or the like is immersed in a solution containing a graft monomer for a predetermined time.

An example of the method for producing a polyurethane or the like includes a step of, while a compound having a choline hydrogen phosphate structure and a functional group that reacts with a radical is brought into contact with a polyurethane or the like, irradiating a surface of the polyurethane or the like with ionizing radiation to generate a radical, thereby forming a graft chain. The ionizing radiation is not particularly limited and is, for example, an electron beam.

Another example of the method for producing a polyurethane or the like includes a step of irradiating a surface of a polyurethane or the like with ionizing radiation to generate a radical, and a step of bringing a compound having a choline hydrogen phosphate structure and a functional group that reacts with a radical into contact with the surface to form a graft chain.

The molecular weight of the polyurethane or the like is not particularly limited, and, for example, the weight-average molecular weight is preferably 5,000 to 500,000, and more preferably 10,000 to 300,000. Herein, the weight-average molecular weight is measured with a GPC apparatus using tetrahydrofuran (THF) as a solvent and is determined as a polystyrene-equivalent value. Specific measurement conditions are as follows.
Column: Polystyrene gel columns manufactured by Tosoh Corporation (TSK gel G4000HXL + TSK gel G3000HXL + TSK gel G2000HXL + TSK gel G1000HXL two columns are connected in series in this order)
Column temperature: 40°C
Detector: Differential refractive index detector (RID-6A, manufactured by Shimadzu Corporation)
Flow rate: 1 mL/min

### <Antithrombogenic Material>

The polyurethane or the like of this embodiment has good biocompatibility, can stably exhibit antithrombogenic performance for a long period, and, in particular, can be effectively used as, for example, materials of medical tools, such as various medical devices and apparatuses, that require blood compatibility, or antithrombogenic coating agents for these medical tools. By using the polyurethane or the like of this embodiment for such purposes, medical devices, apparatuses, and the like that have blood compatibility and that can stably exhibit good antithrombogenic performance can be obtained.

Examples of medical tools, such as medical devices and apparatuses, including the polyurethane or the like of this embodiment include, but are not particularly limited to, hemodialysis membranes, blood plasma separation membranes, adsorbing materials of waste products in the blood, membrane materials (partition between blood and oxygen) for artificial lungs, sheet materials of sheet lungs in artificial heart and lung, main artery balloons, blood bags, catheters, cannulas, shunts, blood circuits, and stents.

When the polyurethane or the like of this embodiment is used as an antithrombogenic coating agent containing the polyurethane or the like, for example, a method including dissolving the polyurethane or the like in an organic solvent, and subsequently applying the solution to a treatment object by an appropriate method such as coating, spraying, or dipping is employed. Examples of the organic solvent include, but are not particularly limited to, tetrahydrofuran (THF), hexamethylphosphoric triamide (HMPA), N-methyl-2-pyrrolidone (NMP), monomethylformamide (NMF), N,N-dimethylformamide (DMF), dimethylacetamide (DMAc), THF-methanol mixtures, THF-ethanol mixtures, and THF-propanol mixtures. The antithrombogenic coating agent may optionally contain, besides the polyurethane or the like of this embodiment, polymer materials that have been used to date as materials of various medical tools that require blood compatibility. Examples of polymer materials include, but are not particularly limited to, polyurethane, polyvinyl chloride, polyester, polypropylene, and polyethylene.

The concentration of the polyurethane or the like in the antithrombogenic coating agent is not particularly limited and can be appropriately determined, according to the type of polyurethane or the like used, within a range in which the polyurethane or the like is soluble in the organic solvent. In the case where the polyurethane or the like of this embodiment is used as a mixture with another polymer, the content ratio of the polyurethane or the like of this embodiment is preferably 1% to 99% by mass, and more preferably 5% to 80% by mass when the total amount of the polyurethane or the like of this embodiment and the other polymer is 100% by mass.

After the antithrombogenic coating agent is applied, the organic solvent is removed to form a coating layer formed of the polyurethane or the like of this embodiment. The method for removing the organic solvent is not particularly limited. For example, a preferred method is a method including drying by heating in an inert gas atmosphere such as nitrogen, argon, or helium at about 20°C to 100°C for about 0.1 to 180 minutes, and subsequently drying under reduced pressure at about 20°C to 100°C for about 0.1 to 36 hours.

The thickness of the coating layer to be formed is not particularly limited, and can be usually about 0.1 to 100 µm, and preferably about 0.5 to 70 µm. The thickness of the coating layer can be easily adjusted by the polymer concentration in the coating composition or the number of times of coating.

The material of a medical tool on which the coating layer is to be formed is not particularly limited, and, in general, the above polymer materials that have been used to date as materials of medical tools are used.

When the polyurethane or the like of this embodiment is used as a material of medical tools, the polyurethane or the like of this embodiment may be used alone or may be used as a mixture with a polymer material that is used as a material of medical tools that require blood compatibility according to, for example, desired physical properties. In the case where the polyurethane or the like is used as a mixture, usually, the content ratio of the polyurethane or the like of this embodiment may be 1% to 60% by mass, or may be 5% to 50% by mass when the total amount of the polyurethane or the like of this embodiment and the other polymer is 100% by mass. To obtain a medical tool using the polyurethane or the like of this embodiment as a material, the medical tool can be produced by a publicly known method that has been employed to date according to the target product.

### EXAMPLES

The present invention will now be more specifically described with reference to Examples, but the present invention is not limited to the following Examples. Note that "part" or "%" in the following Examples and Comparative Examples is based on mass unless otherwise specified.

### <Reagent Used>

### (1) Polyisocyanate

- DURANATE (registered trademark) 50MS (manufactured by Asahi Kasei Corporation) (HDI)
- MILLIONATE MT (manufactured by Tosoh Corporation) (MDI)
- VESTANAT H12-MDI (manufactured by Evonik Industries AG.) (hydrogenated MDI)
- TAKENATE (registered trademark) 500 (manufactured by Mitsui Chemicals, Inc.) (XDI)

### (2) Polyol

- NISSO-PB G-1000 (manufactured by Nippon Soda Co., Ltd.)
- NISSO-PB G-3000 (manufactured by Nippon Soda Co., Ltd.)
- NISSO-PB GI-1000 (manufactured by Nippon Soda Co., Ltd.)
- PTMG-1000 (manufactured by Mitsubishi Chemical Corporation)
- KURARAY POLYOL P-1010 (manufactured by Kuraray Co., Ltd.)
- EXCENOL 1020 (manufactured by AGC Inc.)

### (3) Chain extender/Crosslinking agent

- 1,4-Butanediol (manufactured by Mitsubishi Chemical Corporation)
- Glycerin (manufactured by Sakamoto Yakuhin Kogyo Co., Ltd.)
- JEFFAMINE D-230 (manufactured by Huntsman Corporation)

### (4) Catalyst

- NEOSTANN U-810 (manufactured by Nitto Kasei Co., Ltd.)

### <Example 1>

First, in a four-necked flask equipped with a stirrer, a reflux condenser, a thermometer, and a nitrogen inlet tube, a predetermined amount of DURANATE 50MS serving as a polyisocyanate and a predetermined amount of NISSO-PBG-1000 serving as a polyol were mixed and stirred at 80°C for two hours to obtain a primary reaction product. Subsequently, the primary reaction product was mixed with predetermined amounts of 1,4-butanediol and NEOSTANN U-810, and the mixture was then heat-compressed with a heat press machine and then cured at 80°C for 24 hours to obtain a polyurethane film (film thickness: 100 µm). The mass of the polyurethane film was measured in order to calculate the amount of graft of a monomer later.

Next, one surface of the polyurethane film was irradiated with 200 kGy of an electron beam in a nitrogen atmosphere using a low-energy electron beam (irradiation condition: acceleration voltage 300 kV). In addition, a 20 mass% aqueous solution of a choline hydrogen phosphate 1 represented by structural formula (2) below was prepared as a graft monomer.

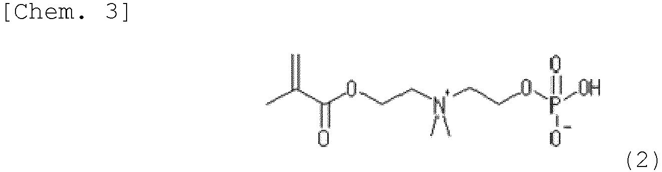

In the preparation of this aqueous solution, pure water from Milli-Q IQ7015 pure water production equipment manufactured by Merck KGaA was used. Subsequently, dissolved oxygen in the aqueous solution was removed in mixing of nitrogen, and the urethane film was then immersed in the solution at 50°C for four hours without being exposed to the air. After immersion, washing was performed with pure water and ethanol to obtain a film A having a graft chain including a choline hydrogen phosphate structure.

### [Synthesis of Choline Hydrogen Phosphate 1]

To a four-necked flask purged with nitrogen, 2-(dimethylamino)ethyl methacrylate (10 mmol), 2-bromoethanol (10 mmol), and dehydrated CH₃CN (20 mL) were added, the resulting solution was then concentrated by reflux at 70°C for 24 hours, and reprecipitation was then performed by adding ethyl acetate to obtain 2-cholinium methacrylate bromide as an intermediate. To a four-necked flask which was purged with nitrogen and the temperature of which was adjusted to 0°C, 2-cholinium methacrylate bromide (10 mmol) and dehydrated CH₃CN (30 mL) were added, and phosphoryl chloride (30 mmol) was added dropwise. Twelve hours later, deionized water (2.8 mL) was added, and stirring was performed for 12 hours. After the resulting solution was concentrated, the crude product was isolated by reverse phase silica gel chromatography to obtain the choline hydrogen phosphate 1.

The mass of the obtained film A was measured and compared with the mass of the polyurethane film before grafting to determine the mass of the graft chain relative to the film A (1.0 g). This mass was divided by the molecular weight (295) of the choline hydrogen phosphate 1 to determine the content (mol) of phosphorus derived from the choline hydrogen phosphate structure relative to the film A (1.0 g).

### <Evaluation of Antithrombogenicity with Film A>

Antithrombogenicity with the film A was evaluated by the following method. Specifically, the film A was cut to a circular shape having a diameter of about 3 cm and attached to the center of a watch glass having a diameter of 10 cm. On this film, 200 µL of sodium citrate plasma of rabbit (Japanese white rabbit) was placed, 200 µL of a 0.025 mol/l aqueous calcium chloride solution was added thereto, and the watch glass was gently shaken so as to mix the liquid while being floated in a constant-temperature bath at 37°C. The time elapsed from the time of addition of the aqueous calcium chloride solution to the coagulation of the plasma (the time at which the plasma does not move) was measured, and this elapsed time was then divided by the time required for coagulation of the plasma when the same operation was performed on glass. This value was used as a coagulation time relative value to evaluate antithrombogenicity. A larger coagulation time relative value means better antithrombogenicity.

### <Evaluation of antithrombogenicity with Film B>

Furthermore, the film A was immersed in a PBS buffer, and elution was performed for two weeks in a constant-temperature bath shaker at 37°C to obtain a film B. The PBS buffer was exchanged daily. Subsequently, antithrombogenicity with the film B was evaluated in the same manner as in the film A. That is, the evaluation of antithrombogenicity with the film B is the same as the evaluation of antithrombogenicity with the film A except that the evaluation of antithrombogenicity with the film B is performed under severer conditions than that of antithrombogenicity with the film A.

### <Example 2>

Example 2 was prepared by the same method as in Example 1 except that, in Example 1, the urethane film was immersed in the 20% aqueous solution of the choline hydrogen phosphate 1 at 50°C for four hours, whereas, in Example 2, the urethane film was immersed for two hours. The ratio of the graft chain varies due to this difference, and the content of phosphorus derived from the structure represented by general formula (1) and contained in 1.0 g of the polymer is thereby changed.

### <Example 3>

Example 3 was prepared by the same method as in Example 1 except that, in Example 1, the urethane film was immersed in the 20% aqueous solution of the choline hydrogen phosphate 1 at 50°C for four hours, whereas, in Example 3, the urethane film was immersed for 30 minutes.

### <Example 4 to Example 13>

Polyurethane films were obtained in the same manner as in Example 1 using the predetermined raw materials shown in tables below.

### <Example 14>

A polyurethane film was obtained in the same manner as in Example 1. Next, a polyurethane film having a graft chain including a choline hydrogen phosphate structure was obtained in the same manner as in Example 1 except that a choline hydrogen phosphate 2 represented by structural formula (3) below was used as a graft monomer.

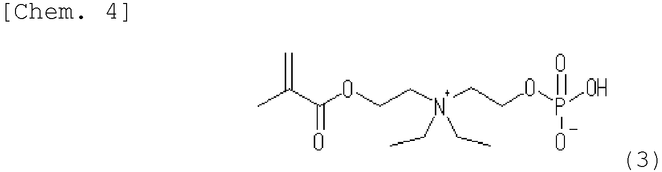

### <Comparative Examples 1 and 2>

Polyurethane films were obtained in the same manner as in Example 1 using the predetermined raw materials shown in the table below. Next, polyurethane films having a graft chain including a phosphorylcholine structure were obtained in the same manner as in Example 1 except that 2-methacryloyloxyethyl phosphorylcholine (manufactured by NOF Corporation) was used as a graft monomer.

### <Comparative Example 3>

With reference to Japanese Patent No. 4042162, an alcohol derivative A represented by structural formula (4) below was synthesized as a substance in which a diol having an OH group derived from phosphoric acid was introduced into the main chain.

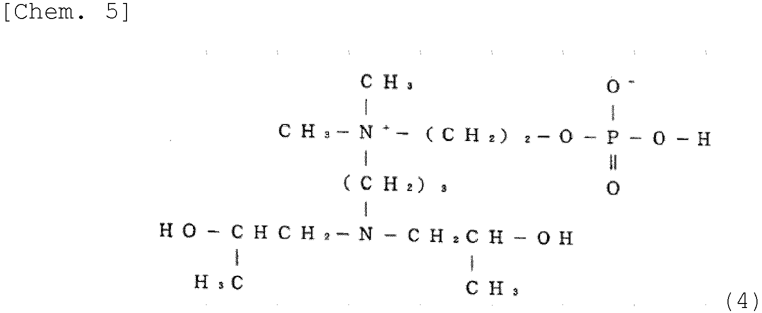

Specifically, equimolar amounts of 2-hydroxy-1,3,2-dioxaphospholane 2-oxide (manufactured by Chemieliva Pharmaceutical Co., Ltd.) (shown by structural formula (5) below) and 4-(3-N,N-dimethylaminopropyl)-4-aza-2,6-dihydroxyheptane were dissolved in dry acetonitrile, and reaction was then conducted in a sealed reactor at 65°C for 24 hours. After the reaction, the solvent was distilled off under reduced pressure, and the residue was washed with cyclohexane several times to obtain an alcohol derivative as an intermediate.

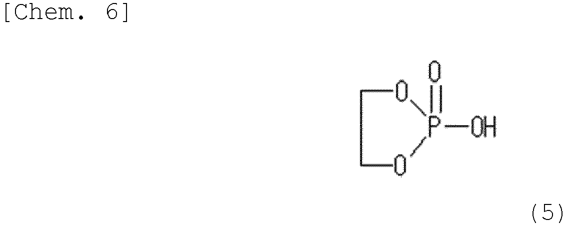

In 100 mL of dimethylacetamide (DMAc), 41 g of the obtained alcohol derivative was dissolved. After the inside of the reactor was sufficiently replaced with argon gas, a solution prepared by dissolving 20 g of DURANATE 50MS in 30 mL of DMAc was slowly added dropwise to the solution. After the dropwise addition, stirring was performed at 100°C for 24 hours to conduct polymerization. This reaction mixture was poured into 1,500 mL of water while stirring, the generated precipitate was filtered out and dissolved in tetrahydrofuran (THF). Subsequently, the resulting solution was further poured into a 50 vol% aqueous methanol solution while stirring, the generated precipitate was collected and dried under reduced pressure to obtain a polymer. This polymer was dissolved in THF to prepare a 5% solution. This solution was uniformly placed on a glass plate, dried at 40°C for eight hours in a nitrogen stream, and then dried under reduced pressure at 40°C for 15 hours to obtain a film having a thickness of about 100 µm.

The following Tables 1 and 2 show blending materials and the amounts of blending in each experiment, and Table 3 shows evaluation results of each experiment.

### [Table 1]

**[Table 1]**

| | | Average molecular weight | Free isocyanate group content (mass%) | Hydroxyl value (mg KOH/g) | Examples 1 to 3 | Example 4 | Example 5 | Example 6 | Example 7 | Example 8 |
|---|---|---|---|---|---|---|---|---|---|---|
| Polyisocyanate | DURANATE 50MS | 168 | 50 | - | 17.12 | | | | 17.72 | 15.98 |
| | MILLIONATE MT | 250 | 33.6 | - | | 23.52 | | | | |
| | VESTANAT H12MDI | 262 | 32.0 | - | | | 24.4 | | | |
| | TAKENATE 500 | 188 | 44.6 | - | | | | 18.8 | | |
| Polyol | NISSO-PB G-1000 | 1400 | - | 73 | 78.3 | 72.3 | 71.4 | 76.73 | 79.05 | 73.1 |
| | NISSO-PB G-3000 | 3000 | - | 33 | | | | | | |
| | NISSO-PB GI-1000 | 1500 | - | 68 | | | | | | |
| | PTMG-1000 | 1000 | - | 112 | | | | | | |
| | KURARAY POLYOL P-1010 | 1000 | - | 112 | | | | | | |
| | EXCENOL 1020 | 1000 | - | 112 | | | | | | |
| | Alcohol derivative A | 342 | - | 328 | | | | | | |
| Chain extender Crosslinking agent | 1,4-Butanediol | 90 | - | 1247 | 4.58 | 4.18 | 4.2 | 4.47 | | |
| | Glycerin | 92 | - | 1830 | | | | | 3.23 | |
| | JEFFAMINE D-230 | 230 | - | 488 | | | | | | 10.92 |
| Catalyst | NEOSTANN U-810 | - | - | - | 0.003 | 0.003 | 0.003 | 0.003 | 0.003 | 0.003 |
| Total | | | - | - | 100 | 100 | 100 | 100 | 100 | 100 |
| NCO index | | - | - | - | 1.0 | 1.0 | 1.0 | 1.0 | 1.0 | 1.0 |

### [Table 2]

**[Table 2]**

| | | Average molecular weight | Free isocyanate group content (mass%) | Hydroxyl value (mg KOH/g) | Example 9 | Example 10 | Example 11 | Example 12 | Example 13 | Example 14 | Comparative Example 1 | Comparative Example 2 | Comparative Example 3 |
|---|---|---|---|---|---|---|---|---|---|---|---|---|---|
| Polyisocyanate | DURANATE 50MS | 168 | 50 | - | 8.8 | 16.2 | 23.6 | 23.6 | 23.6 | 17.12 | 17.12 | | 32.8 |
| | MILLIONATE MT | 250 | 33.6 | - | | | | | | | | 23.52 | |
| | VESTANAT H12MDI | 262 | 32.0 | - | | | | | | | | | |
| | TAKENATE 500 | 188 | 44.6 | - | | | | | | | | | |
| Polyol | NISSO-PB G-1000 | 1400 | - | 73 | | | | | | 78.3 | 78.3 | 72.3 | |
| | NISSO-PB G-3000 | 3000 | - | 33 | 88.85 | | | | | | | | |
| | NISSO-PB GI-1000 | 1500 | - | 68 | | 79.5 | | | | | | | |
| | PTMG-1000 | 1000 | - | 112 | | | 70.2 | | | | | | |
| | KURARAY POLYOL P-1010 | 1000 | - | 112 | | | | 70.2 | | | | | |
| | EXCENOL 1020 | 1000 | - | 112 | | | | | 70.2 | | | | |
| | Alcohol derivative A | 342 | - | 328 | | | | | | | | | 67.2 |
| Chain extender Crosslinking agent | 1,4-Butanediol | 90 | - | 1247 | 2.35 | 4.3 | 6.2 | 6.2 | 6.2 | 4.58 | 4.58 | 4.18 | |
| | Glycerin | 92 | - | 1830 | | | | | | | | | |
| | JEFFAMINE D-230 | 230 | - | 488 | | | | | | | | | |
| Catalyst | NEOSTANN U-810 | - | - | - | 0.003 | 0.003 | 0.003 | 0.003 | 0.003 | 0.003 | 0.003 | 0.003 | |
| Total | | | - | - | 100 | 100 | 100 | 100 | 100 | 100 | 100 | 100 | 100 |
| NCO index | | - | - | - | 1.0 | 1.0 | 1.0 | 1.0 | 1.0 | 1.0 | 1.0 | 1.0 | 1.0 |

### [Table 3]

**[Table 3]**

| | Structure of polyurethane or the like | | | Graft monomer | Phosphorus content (mmol/g) | Film | |
|---|---|---|---|---|---|---|---|
| | Polyisocyanate | Polyol | Chain extender/Crosslinking agent | | | A | B |
| Example 1 | HDI | NISSO-PB G-1000 | 1,4-Butanediol | Choline hydrogen phosphate 1 | 6.4 | 7.3 | 7.0 |
| Example 2 | HDI | NISSO-PB G-1000 | 1,4-Butanediol | Choline hydrogen phosphate 1 | 3.4 | 5.2 | 5.1 |
| Example 3 | HDI | NISSO-PB G-1000 | 1,4-Butanediol | Choline hydrogen phosphate 1 | 1.6 | 4.2 | 4.0 |
| Example 4 | MDI | NISSO-PB G-1000 | 1,4-Butanediol | Choline hydrogen phosphate 1 | 1.5 | 4.1 | 4.0 |
| Example 5 | Hydrogenated MDI | NISSO-PB G-1000 | 1,4-Butanediol | Choline hydrogen phosphate 1 | 3.2 | 5.0 | 4.8 |
| Example 6 | XDI | NISSO-PB G-1000 | 1,4-Butanediol | Choline hydrogen phosphate 1 | 6.1 | 6.4 | 5.8 |
| Example 7 | HDI | NISSO-PB G-1000 | Glycerin | Choline hydrogen phosphate 1 | 6.4 | 7.0 | 6.8 |
| Example 8 | HDI | NISSO-PB G-1000 | JEFFAMINE D-230 | Choline hydrogen phosphate 1 | 6.0 | 6.9 | 6.6 |
| Example 9 | HDI | NISSO-PB G-3000 | 1,4-Butanediol | Choline hydrogen phosphate 1 | 6.3 | 7.4 | 7.1 |
| Example 10 | HDI | NISSO-PB GI-1000 | 1,4-Butanediol | Choline hydrogen phosphate 1 | 6.4 | 6.9 | 6.5 |
| Example 11 | HDI | PTMG-1000 | 1,4-Butanediol | Choline hydrogen phosphate 1 | 6.1 | 6.8 | 6.7 |
| Example 12 | HDI | KURARAY POLYOL P-1010 | 1,4-Butanediol | Choline hydrogen phosphate 1 | 6.1 | 6.6 | 6.3 |
| Example 13 | HDI | EXCENOL 1020 | 1,4-Butanediol | Choline hydrogen phosphate 1 | 3.4 | 4.9 | 4.7 |
| Example 14 | HDI | NISSO-PB G-1000 | 1,4-Butanediol | Choline hydrogen phosphate 2 | 6.2 | 7.1 | 6.9 |
| Comparative Example 1 | HDI | NISSO-PB G-1000 | 1,4-Butanediol | Phosphorylcholine | 3.1 | 2.7 | 2.4 |
| Comparative Example 2 | MDI | NISSO-PB G-1000 | 1,4-Butanediol | Phosphorylcholine | 1.2 | 1.1 | 0.9 |
| Comparative Example 3 | HDI | Alcohol derivative A | - | - | 2.0 | 2.4 | 1.9 |

The results in Table 3 showed the following. Specifically, it was found that Examples each having a graft chain including the structure represented by general formula (1) had larger coagulation time relative values than Comparative Examples in each of the case where the film A was used and the case where the film B was used. That is, Examples were found to exhibit better antithrombogenicity than Comparative Examples.

Furthermore, the comparison of Example 1 to Example 3 showed that, as the phosphorus content increases, that is, as the ratio of the graft chain increases, the coagulation time relative value tends to increase. Consequently, it was found that, as the ratio of the graft chain increases, antithrombogenicity is enhanced.

The present invention is not limited to the above-described embodiments and can be realized in various configurations without departing from the gist thereof. For example, the technical features in the embodiments and Examples corresponding to the technical features in each of the aspects described in the section of Summary of Invention can be replaced or combined as appropriate to solve part or the entirety of the above-described problems or to attain part or the entirety of the above-described advantageous effects. In addition, unless the technical features are described as being essential in this specification, the technical features can be omitted as appropriate.

## Claims

1. A polyurethane or polyurethane urea comprising a graft chain including a structure represented by general formula (1) : 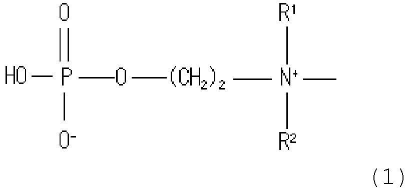 (where R¹ and R² are each an alkyl group having 1 to 20 carbon atoms, an aryl group having 6 to 12 carbon atoms, or an aralkyl group having 7 to 20 carbon atoms and may be the same or different from each other).

2. The polyurethane or polyurethane urea according to Claim 1,
wherein the polyurethane or polyurethane urea contains phosphorus derived from the structure represented by general formula (1) in an amount of 2.0 mmol or more and 10.0 mmol or less relative to 1.0 g of the polymer.

3. The polyurethane or polyurethane urea according to Claim 1 or 2,
wherein R¹ and R² are each an alkyl group having 1 to 20 carbon atoms and may be the same or different from each other.

4. An antithrombogenic coating agent comprising the polyurethane or polyurethane urea according to any one of Claims 1 to 3.

5. An antithrombogenic medical tool comprising the polyurethane or polyurethane urea according to any one of Claims 1 to 3.

6. A method for producing the polyurethane or polyurethane urea according to any one of Claims 1 to 3, the method comprising:
a step of, while a compound having the structure represented by general formula (1) and a functional group that reacts with a radical is brought into contact with a polyurethane or polyurethane urea, irradiating a surface of the polyurethane or polyurethane urea with ionizing radiation to generate a radical, thereby forming a graft chain.

7. A method for producing the polyurethane or polyurethane urea according to any one of Claims 1 to 3, the method comprising:
a step of irradiating a surface of a polyurethane or polyurethane urea with ionizing radiation to generate a radical; and
a step of bringing a compound having the structure represented by general formula (1) and a functional group that reacts with a radical into contact with the surface to form a graft chain.
